# EUROPEAN PATENT SPECIFICATION

(11) **EP 3 706 813 B1**
(45) Date of publication and mention of the grant of the patent: **09.10.2024**
(21) Application number: 18800315.6
(22) Date of filing: 06.11.2018
(51) Int. Cl.: A61L 15/60, A61L 15/46

(54) **ANTIMICROBIAL DRESSING**
ANTIMIKROBIELLER VERBAND
PANSEMENT ANTIMICROBIEN

(30) Priority: 06.11.2017 GB 201718349
(43) Date of publication of application: 16.09.2020
(60) Divisional application: 23195381.1
(73) Proprietor: Brightwake Limited, Nottingham, Nottinghamshire NG17 7JZ (GB)
(72) Inventor: COTTON, Stephen, Nottingham Nottinghamshire NG15 8EQ (GB)
(74) Representative: Adamson Jones
(86) International application number: PCT/GB2018/053219
(87) International publication number: WO 2019/086911

(56) References cited:
- EP-A1- 2 842 580
- EP-A1- 2 898 903
- WO-A1-03/090654
- WO-A1-2007/046806
- GB-A- 2 496 310
- US-A- 4 728 323

## Description

This invention relates to antimicrobial wound dressings.

Wound dressings play an important role in wound care. Different types of wound dressings are required to meet different clinical needs and address different problems.

For example, a wound dressing can play an important part in the prevention and management of wound infection. Infection of wounds during the healing process is major concern in healthcare and, as well as being a source of pain and distress to the patient, can lead to serious complications including tissue loss, systemic infections, septic shock, and death.

One means of combating wound infection is to include an antimicrobial agent in a wound dressing. Silver is recognised as a useful antimicrobial agent and can provide a topical antimicrobial barrier, to reduce the bacteria and fungi counts on and around a wound surface. Many proposals for incorporating silver into wound dressings have been made, including providing the silver in the form of silver fibres, and a variety of means of making such would dressings have been suggested.

WO03090654 discloses conductive wound dressings comprising at least one conductive layer comprising a metal or metal alloy coated fibre. WO2007046806 describes a wound dressing having at least one layer comprising a conformable, conductive substrate comprising one or more yarns, at least one of which comprises a fibre coated with an antimicrobial metal in combination with a non-coated fibre. EP2842580 relates to a silver antimicrobial fibre, fabric and wound dressing, wherein, in the manufacturing process, silver nitrate is added into a spinning polymer solution and fibres are extruded through the wet spinning procedure. US4728323 relates to a wound dressing including a film of silver salt on the surface of a conformable substrate. EP2898903 discloses a wound dressing which is a knitted fabric comprising a fluffy layer of gelling fibres and a layer of non-gelling fibres as a backing, wherein the gelling fibres are knitted onto the backing structure with a length of free fibre lying outside the backing structure. The fibres may comprise silver. GB2496310 describes wound dressings including structure knitted from a yarn comprising a blend of gelling fibres and non-gelling fibres. The wound dressings may comprise silver. WO2013179047 relates to a wound dressing comprising a mat of gel forming fibres comprising silver, and having an open structure reinforced with, for example, textile fibres or threads.

Disclosed herein is a wound dressing comprising an antimicrobial layer, wherein said antimicrobial layer comprises a knitted fabric in which a silver thread is knitted, optionally with a non-metallic thread.

In accordance with the above, the antimicrobial layer may be a knitted fabric consisting of silver thread, or it may be a knitted fabric in which a silver thread and a non-metallic thread are knitted together.

The antimicrobial layer may form a wound-contacting surface of the dressing, or it may be included in a composite dressing between other layers of the dressing.

The non-metallic fibre may be any flexible polymeric material, including, for example, a polyamide, polyester, polyolefin, acrylic, or polypropylene, or it may be a mixture thereof. Alternatively, the non-metallic fibre may be a natural fibre, for example cotton.

Where present, the non-metallic fibre will normally form the warp thread of the fabric. However, it may alternatively form the weft thread of the fabric.

The silver thread, if used in combination with a non-metallic fibre, will normally form the weft thread of the fabric. However, it may alternatively form the warp thread of the fabric.

The silver thread may comprise a thread in which metallic silver is bonded to the surface of a non-metallic base fibre; that is, the thread is a silver-coated fibre. The base fibre may be any flexible polymeric material, including, for example, a polyamide, polyester, polyolefin, acrylic, or polypropylene, or it may be a mixture thereof. The base fibre may also be a natural fibre, for example cotton. Alternatively, the silver thread is a pure silver thread, i.e., a 100% silver thread.

Preferably, in a thread in which metallic silver is bonded to the surface of a non-metallic base fibre, the metallic silver is permanently bonded to the surface of the flexible polymer to coat the entire surface of the base fibre, providing a uniform coating thereon.

When the metallic silver is bonded to the surface of a non-metallic fibre, the silver may be present in at least an amount sufficient to provide a uniform coating on the non-metallic fibre. For example, the silver may be present in a total amount of at least about 3% by weight of the total weight of the silver-coated fibre, such as from 3-15%, 3-14%, 3-13%, 3-12%, 3-11%, 3-10%, 3-9%, 3-8%, 3-7%, 3-6%, 3-5%, 3-4%, for example from 3-7%, 5-10%, or around 3%, 4%, 5%, 6%, 7%, 8%, 9%, 10%, 11%, 12%, 13%, 14% or 15% by weight of the total weight of the silver-coated fibre. Larger amounts of silver may also be used in the coated fibre, for example up to about 20%, or up to about 25%, by weight of the total weight of the silver-coated fibre.

Means for coating base fibres are known. Suitable silver-coated fibres include those manufactured as described in US Patent Number 4,042,737. Suitable fibres are commercially available from Noble Biomaterials, Inc., under the tradename X-STATIC^{®}. X-STATIC^{®} fibres comprise 99.9% pure silver. Thus, a preferred silver coated fibre for use in the present invention is a silver coated fibre wherein the silver coating comprises around 99.9% silver. Preferably the coating provides 100% coverage on the fibre.

In use, silver ions are released from the fibre in a continuous fashion. Because the metallic silver is permanently bonded to the surface of the flexible polymer, metallic silver is not released.

The silver, and if present, the non-metallic thread, may be knitted onto a non-woven sheet of a thermoplastic material.

Knitting of the thread onto a non-woven sheet of thermoplastic material may be achieved by a stitch-bonding process, for example. Stitch-bonding is a known process, in which a flexible substrate, such as a nonwoven fabric, is pierced with a series of pointed needles positioned adjacent to each other. These needles then stitch a network of textile yarns into the substrate.

Knitting of the silver, and if present, the non-metallic threads onto a non-woven sheet of a thermoplastic material allows the antimicrobial layer to be fused onto another component of the dressing, such as to an absorbent material, to form a composite wound dressing. Said composite wound dressing comprises an antimicrobial layer and an absorbent material, wherein said antimicrobial layer comprises a knitted fabric in which a silver thread is knitted, optionally with a non-metallic thread. Fusing of the antimicrobial layer onto an absorbent base in this manner provides a dressing of high integrity which is resistant to loss of fibre due to fraying of the knitted fabric. Fusing is achieved by applying heat to an assembly of desired components, including the antimicrobial layer and the thermoplastic material.

The disclosed composite wound dressing can thus be described as comprising an antimicrobial layer and an absorbent material, wherein said antimicrobial layer comprises a silver thread and optionally a non-metallic thread knitted onto a non-woven sheet of a thermoplastic material, and said composite wound dressing comprises a fused assembly of said antimicrobial layer and absorbent material.

The antimicrobial layer may be fused onto multiple components of the dressing, as appropriate. However, a dressing in accordance with the present disclosure may comprise only a single antimicrobial layer which is a knitted silver thread or a knitted layer of a silver thread and a non-metallic thread, optionally fused onto an absorbent material.

Thermoplastic materials are well known to the person skilled in the art and include, for example, polyamides, polyesters, polyolefins, acrylics, polypropylenes, and mixtures thereof.

Dressings in accordance with the present disclosure may comprise gelling fibres.

Accordingly, the present invention provides a wound dressing comprising an antimicrobial layer, wherein said antimicrobial layer is a fabric comprising a spun yarn, which yarn comprises a blend of gelling fibres, non-gelling fibres, and silver fibres comprising metallic silver bonded to the surface of a non-metallic fibre.

Gelling fibres are known for use in wound dressings. They have a greater capacity for absorbing liquid than standard textile fibres and, on absorbing liquid, they become moist and slippery. This prevents the dressing from adhering to the wound and therefore makes removal of the dressing easier. When gelling fibres are blended with non-gelling fibres in a yarn, a knitted fabric of high structural integrity can be produced, which fabric has the advantages of high structural integrity and excellent capacity for absorbing wound exudate. WO 2013/064831 describes such a knitted fabric.

Methods for producing yarn from blended fibres are known in the art. The yarn for use in the present invention may be spun for example from a blend of gelling fibres, non-gelling fibres and silver fibres, by any known method.

A variety of techniques for producing a fabric from a spun yarn (for example, by knitting) are known in the art, and are suitable for use in the present invention.

The gelling fibres may be any suitable gelling fibres known in the art, including pectin fibres, alginate fibres, fibres made from alginate and another polysaccharide, chitosan fibres, hyaluronic acid fibres, fibres of other polysaccharides or derived from gums, or chemically-modified cellulosic fibres, e.g., carboxymethyl cellulose (CMC). The gelling fibres may be a combination or blend of different gelling fibres. A particularly preferred gelling fibre is CMC.

The non-gelling fibres may be any suitable non-gelling fibres known in the art, or mixtures thereof. Suitable fibres included textile fibres, and may be natural, e.g., cotton, natural fibres which have been modified, e.g., cellulosic fibres such as viscose or lyocell (e.g., sold under the trade name TENCEL^{®}), or synthetic fibres, e.g. polyester, polypropylene, polyolefin, acrylic, or polyamide. Preferably, the non-gelling fibres are cellulosic fibres, most preferably TENCEL^{®} fibres.

A combination of CMC fibres, TENCEL^{®} fibres, and silver fibres is preferred.

Dressings in accordance with the present invention may comprise an absorbent material. Suitable absorbents include foam materials. Foam dressings are a common type of wound dressing and are known to be particularly useful in dressing chronic wounds that have high levels of exudate.

The foam material may be any suitable foam material known in the art. Typically, the foam is an open-celled foam. Preferably, the foam is a hydrophilic foam. More preferably, the hydrophilic foam is a polyurethane foam. Most preferably, the foam is an open-celled polyurethane foam. The foam typically has a thickness of from 0.5mm to 10mm, for example from 1mm to 7mm, or from 2mm to 7mm, such as 2mm, 3mm, 4mm, 5mm or 6mm. "Thickness" refers to the foam in a natural, uncompressed state.

Other forms of absorbent body include absorbent polymeric materials. Suitable polymeric materials include polysaccharides and polysaccharide derivatives. For example, the absorbent material may be an alginate (i.e., a salt of alginic acid) and in particular sodium alginate or calcium alginate, or a blend of the two. Other suitable absorbent materials include cellulose, a cellulose derivative such as hydroxyethyl cellulose or hydroxypropyl cellulose, and pectin or a pectin derivative such as amidated pectin.

Alternatively, the absorbent material may be of the type commonly referred to as a "superabsorber" or "superabsorbent material". Such materials are typically capable of absorbing many times their own mass of water (e.g., up to 200, 300, 400, 500 or more times their own mass of water). Preferred superabsorbent materials are polymeric superabsorbent materials and include polyacrylate (i.e., a salt of polyacrylic acid), polyacrylamide copolymers, ethylene maleic anhydride copolymer, carboxymethylcellulose, polyvinylalcohol copolymers, polyethylene oxide and starch-grafted copolymers of polyacrylonitrile.

A layered dressing may comprise more than one of absorbent material, each layer being the same or different. In this case, the antimicrobial layer may be disposed between layers of absorbent material, and/or at the wound-facing surface of the dressing.

Dressings in accordance with the present invention may include a hydrophobic gel. Means of applying a hydrophobic gel are known. Generally, a curable hydrophobic gel precursor is applied to the dressing, and caused or allowed to cure, thereby forming a layer of hydrophobic gel.

The hydrophobic gel may be disposed on the wound-contacting surface of the dressing and may coat all or part of the wound-contacting surface of the dressing. For example, the hydrophobic gel may form a border around the edge of the dressing.

Alternatively, or additionally, the hydrophobic gel may impregnate the antimicrobial layer of the dressing.

A preferred hydrophobic gel is a silicone gel, such as a soft silicone gel. Soft silicone is used in wound dressings because it adheres readily to dry skin but does not stick to the surface of a moist wound and does not cause damage on removal. It is therefore particularly useful for use in atraumatic wound dressings. There are several other intrinsic properties of soft silicone that make it particularly advantageous for use in wound dressings. These properties are well documented and include the fact that silicones are non-toxic, non-allergenic and non-sensitising, they do not shed particles or fibres into the wound, they feel soft on the skin and they are comfortable, yet robust.

Suitable silicone gels are formed by reaction between two components that are mixed immediately prior to application to the composite wound dressing. Suitable components that are intended for such reactions to form a silicone gel are readily available commercially. Typically, the two components are a vinyl-substituted silicone and a hydride-containing silicone, such as are known in the art.

Gels having different properties may be produced by varying the proportions and/or nature of the components used in the reaction. For example, the molecular weights of the various components and/or their degree of substitution by reactive groups may be different.

The hydrophobic gel may be coated onto the composite wound dressing at a wide variety of coating weights. The most appropriate coating weight will depend on the properties of the gel and its intended application. Typically, the gel may be coated onto the wound facing surface of the present dressings at a weight of from 50g/m² and 800g/m². The thickness of the gel may typically be between 5µm and 10mm, for example between 20µm and 5mm, or between 0.5mm and 5mm, or between 0.5mm and 2mm, e.g., about 1mm or about 1.5mm.

As hydrophobic gel layers do not permit the free transmission of fluids, wound dressings having a skin-contacting layer coated with hydrophobic gel generally require an opening in the gel layer to allow the transmission of wound exudate away from the wound. Advantageously, the hydrophobic gel layer may be perforated. Perforated hydrophobic gel layers are of particular advantage for use as skin-contacting layers in wound dressings that are in prolonged contact with the skin. The introduction of perforations in the hydrophobic gel layer allows the transmission of fluids, such as water vapour, improving the breathability of the gel layer and thereby improving comfort. The improved breathability of the hydrophobic gel layer allows the entire skin-contacting surface of a dressing to be coated.

Means for introducing perforations into a hydrophobic gel layer are known. For example, WO 2010/061228 describes a method for introducing perforations into a sheet of laminated material that includes a substrate and a layer of hydrophobic gel, which method involves contacting perforating elements with the sheet and subjecting the sheet, at least in the regions contacted with the perforating elements, to high frequency mechanical vibrations. The substrate may form part of a finished product in which the perforated laminate is incorporated, or it may be a processing aid used to facilitate production of the perforated laminate, which is removed prior to incorporation of the laminate into a composite product.

Perforations may be varied considerably in size and shape but are typically circular and between 0.1mm and 5mm, more commonly between 0.5mm and 2mm, in diameter, although smaller and larger perforations are possible.

Typically, perforations in any given product will all be of similar form, although it is possible for a variety of sizes and shapes of perforation to be present in a single product.

The perforations are preferably arranged in a regular array, the perforations typically being separated by 0.2 to 10mm. Most commonly, the number of perforations per unit area is between 1 and 100, more commonly between 1 and 50, or between 1 and 20, perforations/cm². The perforations typically account for more than 5%, and up to 75%, or up to 50%, or up to 25%, of the area of the hydrophobic gel layer.

Wound dressings in accordance with the present invention may be provided in a number of different forms. Wound dressings may consist of, or include, the antimicrobial layer described herein.

For example, dressings in accordance with the present invention may be provided as simple dressings which may be in ribbon or tape form, or may be square, rectangular, circular, ovoid, or may have any other suitable shape, for example to conform to the shape of a specific part of the body, such as the sacrum or the heel. They may also take the form a jointed dressing, that is, a dressing adapted for application to a jointed limb.

Dressings in accordance with the present invention may be provided with a backing layer on the non-wound-facing surface of the dressing. Any suitable material known in the art may be used for the backing layer. For example, the backing layer may be a plastics film such as a polyurethane foam. The backing layer may be attached to the composite wound dressing by means of an adhesive provided on the dressing-facing surface of the backing layer or on the backing layer-facing surface of the dressing.

The backing layer may be oversized, extending beyond the edges of the rest of the wound dressing, for example on all sides to provide a border therearound. In this case, the adhesive may extend over the full surface of the backing layer and may serve to adhere the wound dressings to a patient's skin, around the wound. Suitable skin contact adhesives are known. For example, the skin contact adhesive may be a hydrophobic gel such as a silicone gel, as described above, or an acrylic adhesive, or a combination of the two.

Dressings in accordance with the present invention may be provided on the wound-facing surface with a release liner that is removed to expose the wound-facing surface of the dressing immediately prior to use. Preferably, the release liner is formed in such a way as to be readily grasped and removed, for example by having one or more projecting tabs.

The release line may be formed from any suitable material, for example, form high density polyethylene.

The release liner may be oversized, for example in correspondence with the backing layer.

Dressings in accordance with the present invention may be included in a system for negative pressure wound therapy.

Negative pressure wound therapy (NPWT) involves the application of a pressure that is reduce relative to that of the surroundings (commonly referred to as "negative pressure") to a wound, which causes mechanical contraction of the wound and removal of wound fluid, thus promoting formation of granulation tissue and accelerating wound closure. This technique is particularly effective in the treatment of slow healing wounds such as chronic leg ulcers and large open wounds. A dressing comprising an occlusive drape, traversed by a drainage tube, is applied to the wound opening, forming a seal under which a negative pressure can be established. The drainage tube is connected to a negative pressure source allowing the wound fluid to be drawn away.

The present invention is clearly directed to a wound dressing and the skilled person will be well aware of the general properties and specific nature of materials suitable for use in this particular application.

### EXAMPLE

Dressings in accordance with the present invention, including an antimicrobial layer fused to an absorbent foam, were tested for capability to kill organisms upon contact.

The inhibitory activity of the material was tested against *Staphylococcus aureus* (ATCC 6538) and *Pseudomonas aeruginosa* (ATCC 9027).

### Procedure

Tryptone Soya Agar (TSA) plates were seeded with approximately 10⁴ cfu/ml of the bacteria.

Small pieces of the dressing were added to each TSA plate. In addition, control samples were prepared using small pieces of non-antimicrobial based dressings. These were added to each type of seeded agar plate.

At intervals of 1, 4 and 24 hours, the inhibitory material was removed from the surface of the appropriate seeded plate. The plates were incubated at 30°C (+/-2°C) for a minimum period of 24 hours. This was used to establish whether dressing contact time had any effect on the inhibitory capability of the material. Any leaching of the inhibitory substance from the dressing into the agar, causing inhibition away from the dressing, was also assessed.

### Results

At each time interval, almost complete inhibition was observed for both organisms.

Leaching was observed for *Staphylococcus aureus* at 1 and 4 hours, with extensive leaching at 24 hours.

No leaching was observed for *Pseudomonas aeruginosa.*

### Conclusion

Anti-microbial effect was observed against both organisms in comparison to control samples, particularly after 24 hours. The material was found to be very effective at inhibiting organisms. Extensive leaching of the anti-microbial substance was also seen with *Staphylococcus aureus.*

## Claims

1. A wound dressing comprising an antimicrobial layer, wherein said antimicrobial layer is a fabric comprising a spun yarn, which yarn comprises a blend of
(i) gelling-fibres,
(ii) non-gelling-fibres, and
(iii) silver fibres comprising metallic silver bonded to the surface of a non-metallic fibre.

2. A wound dressing as claimed in claim 1, further comprising an absorbent material selected from an absorbent foam, an absorbent polymeric material, and a superabsorbent material.

3. A wound dressing as claimed in claim 1 or claim 2, further comprising a hydrophobic gel.

4. A wound dressing as claimed in claim 3, wherein the hydrophobic gel is a soft silicone gel.

5. A wound dressing as claimed in claim 3 or claim 4, wherein the hydrophobic gel is perforated.

6. A wound dressing as claimed in any one of claims 3-5, wherein the hydrophobic gel is provided as a coating on a wound-contacting surface of the dressing.

7. A wound dressing as claimed in any one of claims 3-6, wherein the hydrophobic gel impregnates the antimicrobial layer of the dressing.

8. A wound dressing as claimed in any one of the preceding claims, further comprising a backing layer secured to the non-wound-contacting surface of the dressing.

9. A wound dressing as claimed in claim 8, wherein the backing layer extends beyond the antimicrobial layer or the antimicrobial layer and absorber on all sides, to form a border around the wound dressing and is optionally provided with an adhesive for securing the dressing to a patient.

10. A wound dressing as claimed in any one of the preceding claims, further comprising a release liner on the wound-facing surface of the dressing.

11. A system for negative pressure wound therapy, including a wound dressing according to any one of claims 1-7.

## Patentansprüche

1. Wundverband, eine antimikrobielle Schicht umfassend, wobei die antimikrobielle Schicht ein Gewebe ist, das ein gesponnenes Garn umfasst, wobei das Garn eine Mischung umfasst aus
(i) gelierenden Fasern,
(ii) nicht gelierenden Fasern und
(iii) Silberfasern, die metallisches Silber umfassen, das an die Oberfläche einer nicht metallischen Faser gebunden ist.

2. Wundverband nach Anspruch 1, ferner ein absorbierendes Material umfassend, das aus einem absorbierenden Schaum, einem absorbierenden Polymermaterial und einem superabsorbierenden Material ausgewählt ist.

3. Wundverband nach Anspruch 1 oder Anspruch 2, ferner ein hydrophobes Gel umfassend.

4. Wundverband nach Anspruch 3, wobei das hydrophobe Gel ein weiches Silikongel ist.

5. Wundverband nach Anspruch 3 oder Anspruch 4, wobei das hydrophobe Gel perforiert ist.

6. Wundverband nach einem der Ansprüche 3-5, wobei das hydrophobe Gel als eine Beschichtung auf einer mit der Wunde in Kontakt kommenden Oberfläche des Verbands bereitgestellt ist.

7. Wundverband nach einem der Ansprüche 3-6, wobei das hydrophobe Gel die antimikrobielle Schicht des Verbandes durchdringt.

8. Wundverband nach einem der vorhergehenden Ansprüche, ferner eine Trägerschicht umfassend, die an der Oberfläche des Verbands befestigt ist, die nicht mit der Wunde in Kontakt kommt.

9. Wundverband nach Anspruch 8, wobei die Trägerschicht über die antimikrobielle Schicht oder die antimikrobielle Schicht und den Absorber auf allen Seiten hinausragt, um einen Rand um den Wundverband zu bilden, und wobei sie optional mit einem Klebstoff zum Befestigen des Verbands an einem Patienten versehen ist.

10. Wundverband nach einem der vorhergehenden Ansprüche, ferner eine Trennfolie auf der der Wunde zugewandten Oberfläche des Verbands umfassend.

11. System zur Unterdruck-Wundtherapie, einen Wundverband nach einem der Ansprüche 1-7 beinhaltend.

## Revendications

1. Pansement pour plaie comprenant une couche antimicrobienne, ladite couche antimicrobienne étant un tissu comprenant un fil filé, lequel fil comprend un mélange de
(i) fibres gélifiantes,
(ii) fibres non gélifiantes, et
(iii) fibres d'argent comprenant de l'argent métallique lié à la surface d'une fibre non métallique.

2. Pansement pour plaie selon la revendication 1, comprenant en outre un matériau absorbant choisi parmi une mousse absorbante, un matériau polymère absorbant et un matériau superabsorbant.

3. Pansement pour plaie selon la revendication 1 ou la revendication 2, comprenant en outre un gel hydrophobe.

4. Pansement pour plaie selon la revendication 3, ledit gel hydrophobe étant un gel de silicone souple.

5. Pansement pour plaie selon la revendication 3 ou la revendication 4, ledit gel hydrophobe étant perforé.

6. Pansement pour plaie selon l'une quelconque des revendications 3 à 5, ledit gel hydrophobe étant fourni sous la forme d'un revêtement sur une surface du pansement en contact avec la plaie.

7. Pansement pour plaie selon l'une quelconque des revendications 3 à 6, ledit gel hydrophobe imprégnant la couche antimicrobienne du pansement.

8. Pansement pour plaie selon l'une quelconque des revendications précédentes, comprenant en outre une couche support fixée sur la surface du pansement non en contact avec la plaie.

9. Pansement selon la revendication 8, ladite couche support s'étendant au-delà de la couche antimicrobienne ou de la couche antimicrobienne et de l'absorbant de tous les côtés, de manière à former une bordure autour du pansement pour plaie et étant éventuellement dotée d'un adhésif pour fixer le pansement sur un patient.

10. Pansement pour plaie selon l'une quelconque des revendications précédentes, comprenant en outre une doublure antiadhésive sur la surface du pansement faisant face à la plaie.

11. Système pour le traitement des plaies par pression négative, comprenant un pansement pour plaie selon l'une quelconque des revendications 1 à 7.
